(19) Europäisches Patentamt
European Patent Office
Office européen des brevets

(11) EP 0 538 433 B1

(12) # EUROPÄISCHE PATENTSCHRIFT

(45) Veröffentlichungstag und Bekanntmachung des Hinweises auf die Patenterteilung:
**21.04.2004 Patentblatt 2004/17**

(21) Anmeldenummer: **92909703.8**

(22) Anmeldetag: **14.05.1992**

(51) Int Cl.[7]: **A61K 31/48**, A61K 9/70

(86) Internationale Anmeldenummer:
**PCT/DE1992/000400**

(87) Internationale Veröffentlichungsnummer:
**WO 1992/020339 (26.11.1992 Gazette 1992/29)**

(54) **MITTEL ZUR TRANSDERMALEN APPLIKATION ENTHALTEND ERGOLIN-DERIVATE**

AGENT SUITABLE FOR TRANSDERMAL ADMINISTRATION CONTAINING ERGOLIN DERIVATIVES

AGENT POUR ADMINISTRATION TRANSDERMIQUE RENFERMANT DES DERIVES DE L'ERGOLINE

(84) Benannte Vertragsstaaten:
**AT BE CH DE DK ES FR GB GR IT LI LU NL SE**

(30) Priorität: **18.05.1991 DE 4116912**

(43) Veröffentlichungstag der Anmeldung:
**28.04.1993 Patentblatt 1993/17**

(60) Teilanmeldung:
**00250117.9 / 1 027 889**

(73) Patentinhaber: **NeuroBiotec GmbH**
**10115 Berlin (DE)**

(72) Erfinder:
- **RIEDL, Jutta**
  **D-1000 Berlin 19 (DE)**
- **GÜNTHER, Clemens**
  **D-1000 Berlin 65 (DE)**
- **LIPP, Ralph**
  **D-1000 Berlin 31 (DE)**

(74) Vertreter: **Wablat, Wolfgang, Dr. Dr.**
**Wablat . Lange . Karthaus**
**Anwaltssozietät**
**Potsdamer Chaussee 48**
**14129 Berlin (DE)**

(56) Entgegenhaltungen:
**EP-A- 0 137 278** **EP-A- 0 155 229**
**WO-A-91/00746**

- **Dialog Information Services & Base Embase, Embase Nr. 92118765, J.K.C. TSUI: "Future treatment of Parkinson's disease", & CAN. J. NEUROL. SCI. (CANADA), 1992, 19/1 SUPPL. (160-162), siehe Zusammenfassung**
- **Dialog Information Services & Base Embase, Embase Nr. 91321379, A. AGNOLI et al.: "Dopamine receptor agonists as possible first-choice therapy of early Parkinson's disease", & NEW TRENDS CLIN. NEUROPHARMACOL. (ITALY), 1991, 5/2 (41-48), siehe Zusammenfassung**

Bemerkungen:
Die Akte enthält technische Angaben, die nach dem Eingang der Anmeldung eingereicht wurden und die nicht in dieser Patentschrift enthalten sind.



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die Erfindung betrifft ein Mittel zur transdermalen Applikation, welches dadurch gekennzeichnet ist, daß es Ergolin-Derivate gegebenenfalls in Kombination mit einem oder mehreren penetrationsverstärkenden Mittel(n) enthält.
Ergolin-Derivate, wie das bereits seit 1960 bekannte Lisurid [3-(9,10-Dihydro-6-methyl-8α-ergolinyl-1,1-diethylharnstoff] und dessen Derivate, die unter anderen in der DE-A 2 238 540, der EP-A 0021206, der EP-A 0056358 und der EP-A 0160840 beschrieben sind, sind pharmakologisch wirksame Substanzen, die zur Herstellung von Arzneimitteln verwendet werden können.
Derartige Ergolin-Derivate sind beispielsweise solche der allgemeinen Formel

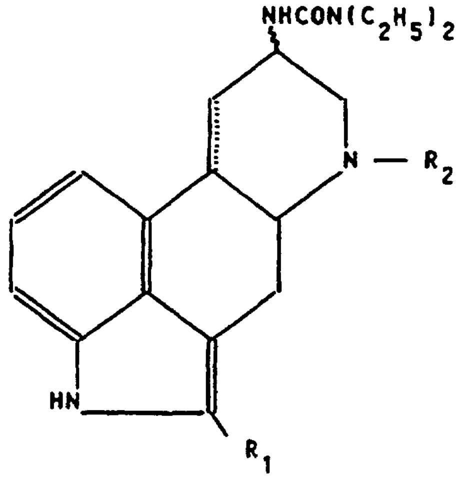

worin

..... eine Einfachbindung oder eine Doppelbindung symbolisiert.
$R_1$ ein Wasserstoffatom, ein Halogenatom oder eine Alkylthiogruppe mit 1 bis 4 Kohlenstoffatomen im Alkylrest darstellt und
$R_2$ eine Alkylgruppe mit 1 bis 4 Kohlenstoffatomen bedeutet, oder deren Salz mit einer physiologisch unbedenklichen Säure.

Als hochwirksame Ergolin-Derivate seien neben dem Lisurid das 8romlisurid [=3-(2-Brom-9,10-dehydro-6-methyl-8α-ergolinyl-1,1-diethylharnstoff], das Tergurid [=3-(6-Methyl-8α-ergolinyl-1,1-diethylharnstoff] und das Protergurid [=3-(6-Propyl-8α-ergolinyl)-1,1-diethylharnstoff] erwähnt.
Als Salze dieser Ergolin-Derivate seien die Sulfate, Phosphate, Maleate, Citrate oder Succinate erwähnt.
Ergolin-Derivate haben heute als Dopaminagonisten einen festen Platz in der Therapie verschiedenster Erkrankungen, die durch Hyperprolaktinämie bedingt sind oder bei denen Prolaktin von pathogenetischer Bedeutung ist.
Für primäres und sekundäres Abstillen sind Dopaminagonisten die Mittel der Wahl, ebenso für die mit Hyperprolaktinämie eingehenden Fertilitätsstörungen der Frau. Auch durch Hyperprolaktinämie bedingte Potenzstörungen des Mannes können erfolgreich behandelt werden. Während Dopamin und Dopaminagonisten beim Gesunden die Sekretion von STH stimulieren, haben sie bei der Akromegalie den gegenteiligen Effekt. Praemenstruelles Syndrom, Praeklampsie, Geriatrie, Mastodynie werden ebenfalls mit dem weiten Wirkungsspektrum von Prolaktin in Verbindung gebracht und können mit Dopaminagonisten behandelt werden. Dem Parkinsonismus liegt ein Dopaminmangel zugrunde, deshalb können Parkinsonpatienten erfolgreich mit Oopaminagonisten behandelt werden.
Viele Ergolin-Derivate, wie zum Beispiel das Lisurid selbst haben nur eine kurze terminale Halbwertzeit und es ist demzufolge schwierig über einen längeren Zeitraum hin im Blut konstante Plasmaspiegel des Medikaments zu erreichen.
Es wurde nun gefunden, daß Ergolin-Derivate gegebenenfalls in Kombination mit einem oder mehreren penetrationsverstärkenden Mittel(n) sehr gut zur Herstellung eines Mittels für die transdermale Applikation des Wirkstoffes verwendet werden kann.
Transdermal zu applizierende Arzneimittel haben bekanntlich den Vorzug. daß sie über einen längeren Zeitraum hin eine gleichmäßigere Freisetzung des Wirkstoffs ermöglichen, als dies in der Regel bei anders - wie zum Beispiel oral - zu applizierenden Mitteln möglich ist. Diese Eigenschaften lassen sich in einer Reihe von Erkrankungen

vorteilhaft ausnutzen. Für in üblichen Pflastermassen schwer lösliche Wirkstoffe, wie zum Beispiel die Ergolin-Derivate ist es aber in der Regel recht problematisch, transdermale Systeme zu erstellen, die eine zur Therapie ausreichende Penetration des Wirkstoffs durch die Haut gewährleisten.

Es wurde nun gefunden, daß es mit Hilfe des erfindungsgemäßen Mittels überraschenderweise möglich ist, eine therapeutisch ausreichende und sehr gleichmäßige Penetrationsgeschwindigkeit der Ergolin-Derivate durch die Haut zu erzielen.

Zur Herstellung pharmazeutischer Präparate kann der Wirkstoff in geeigneten flüchtigen Lösungsmitteln und/oder penetrationsverstärkenden Mitteln gelöst oder suspendiert werden. Die erhaltenen Lösungen oder Suspensionen können mit den üblichen Hilfsstoffen, wie zum Beispiel Verdickungsmitteln versetzt werden. Zu erwähnen ist ferner, daß die erfindungsgemäßen Lösungen oder Suspensionen beispielsweise mittels Siliconelastomeren zu wirkstoffhaltigen Pflastern oder Bandagen verarbeitet werden können (DE-A 31 31 610; US-A 3,996,934 oder US-A 4,336,243).

Geeignete flüchtige Lösungsmittel sind beispielsweise niedere Alkohole, Ketone oder niedere Carbonsäureester wie Ethanol, Isopropanol, Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Geeignete penetrationsverstärkende Mittel sind 1,2-Propandiol und Laurylalkohol. Eine sehr gleichmäßige Applikation mit eingestellter Dosierung des Wirkstoffes kann man erzielen wenn man den Wirkstoff in ein transdermales therapeutisches System (TTS) einbettet. Geeignete transdermale therapeutische Systeme sind solche, die man üblicherweise zur percutanen Applikation von Wirkstoffen anwendet (Yie W. Chien: "Tansdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Reserch 1030 Hamilton Court Menlo Park CA 94025 (415) 328-2228).

So kann man beispielsweise ein solches transdermales therapeutisches System verwenden, welches aus

a) einer undurchlässigen Deckschicht.
einer an der Deckschicht haftenden, das Ergolin-Derivat und gegebenenfalls das oder die penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
einer abziehbaren Schutzschicht, oder

b) einer undurchlässigen Deckschicht,
einem an oder in der Deckschicht befindlichen, das Ergolin-Derivat gegebenenfalls penetrationsverstärkende Mittel enthaltenden Arzneimittelreservoir,
für diese Komponenten durchlässigen Polymerschicht.
einer durchlässigen gegebenenfalls penetrationsverstärkende Mittel enthaltende Hauthaftkleberschicht und
einer abziehbaren Schutzschicht besteht.

Ein transdermales therapeutisches System gemäß Variante a stellt ein einfaches Matrixsystem dar. Es kann beispielsweise wie folgt hergestellt werden.

Eine Lösung oder Suspension von 1 bis 25 Gew. % Wirkstoff, 0-40 Gew. % eines penetrationsverstärkenden Mittels, 30-70 Gew. % eines medizinisch üblichen Klebers aufgefüllt mit einem geeigneten flüchtigen Lösungsmittels zu 100 Gew. % wird auf eine plane undurchlässige Deckschicht gestrichen und nach dem Trocknen mit einer abziehbaren Schutzschicht versehen.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusätzlich mit einem Hauthaftkleber abdecken oder umgeben.

Geeignete Lösungsmittel und penetrationsverstärkenden Mittel sind beispielsweise die bereits erwähnten Flüssigkeiten dieser Art. Als medizinisch übliche Kleber eignen sich Polyacrylate.

Als Schutzschichten eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielsweise 10 bis 100 µm dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise über eine Fläche von 5 bis 100 $cm^2$.

Ein transdermales therapeutisches System gemäß Variante b kann beispielsweise wie folgt hergestellt werden.

Eine undurchlässige Folie wird durch Wärme und/oder Zug so verformt, daß eine 0,1 bis 3 ml fassende Ausbuchtung entsteht. Diese wird mit einer wirkstoffhaltigen Lösung oder Suspension enthaltend 1-50 Gew. % Wirkstoff in einem penetrationsverstärkenden Mittel gefüllt. Die wirkstoffhaltige Lösung oder Suspension kann auch mit bis zu 10 Gew. % Matrixbildner verdickt sein.

Als Abdeckung des Reservoirs zur Haut hin dient eine aufgeschweißte oder aufgeklebte durchlässige Polymerschicht, auf welche eine durchlässige Hauthaftkleberschicht und eine abziehbare Schutzschicht angebracht wird.

Es können bei diesem System die oben erwähnten penetrationsverstärkenden Mittel angewendet werden. Als durchlässige Polymerschicht wird beispielsweise eine 20 bis 200 µm dicke Folie aus Celluloseestern, Celluloseethern. Siliconen oder Polyolefinverbindungen verwendet. Durch Variation dieser Polymerschicht läßt sich die Diffusionsgeschwindigkeit des Wirkstoffes oder Wirkstoffgemisches innerhalb weiter Grenzen variieren.

Als Kleber und Schutzschicht eignen sich die gleichen Materialien, die bei dem transdermalen therapeutischen System gemäß Variante a beschrieben sind.

So lassen sich durch einfache Variation der verschiedenen Parameter transdermale therapeutische Systeme mit unterschiedlichen Freisetzungsraten des Wirkstoffes oder Wirkstoffgemisches herstellen, die zwecks Lagerung beispielsweise in Aluminiumfolien verpackt werden können.

Die Konzentration, in welcher das Ergolin-Derivat optimalerweise in der Penetrationsverstärkung gelöst oder suspendiert wird, ist naturgemäß von der Art des verwendeten Wirkstoffs und Penetrationsverstärker und der angestrebten Einzeldosis abhängig. Sie muß im Einzelfalle mittels der dem Fachmann geläufigen Vorversuche, wie zum Beispiel der Bestimmung der erreichbaren Blutplasmakonzentrationen an Wirkstoff bei ausgewählten erfindungsgemäßen Mitteln pro Fläche ermittelt werden. Im allgemeinen werden Wirkstoffkonzentrationen von 0,2 bis 20 Gewichtsprozent im erfindungsgemäßen Mittel ausreichend sein.

Die Bestimmung des Ausmaßes der Geschwindigkeit der percutanen Resorption durch die erfindungsgemäßen Mittel kann beispielsweise mittels radioaktiv markierter Ergolin-Derivate erfolgen.

Frisch bereitete, von subcutanem Fett befreite Haut vom Abdomen haarloser Mäuse wird in eine Franz Diffusionszelle eingespannt, die als Auffangflüssigkeit isotonische Polyethylenglykol-(MG 400)-Lösung oder Phosphat-Puffer-Lösung vom pH 7 enthält. Dann gibt man 2 µl Testlösung auf die Haut und ermittelt nach 24, 48 und 72 Stunden mittels Flüssigkeits-Scintilationszählung den Gehalt des in die Auffangflüssigkeit gelangten Ergolin-Derivats.

Getestet wurde eine 5 gewichtsprozentige Lösung des Ergolins in Propylenglycol (PG) und Propylenglycol-Laurinsäure 9:1 w/w (PG + 10 % LS).

Die nachfolgende Tabelle zeigt die in diesem Versuch erhaltenen Ergebnisse:

Tabelle

| Parameter | Dimension | Lisurid in | |
|---|---|---|---|
| | | PG | + 10 % LS |
| Maximal-Fluß | µg/cm2/h | 4.0 | 14.6 |
| t (Maximal-Fluß) | h | 15,1 | 4,5 |
| mittlerer Fluß (0-24h) | µg/cm2/h | 3,0 | 7,0 |
| mittlerer Fluß (24-48h) | µg/cm2/h | 1,6 | 4,3 |
| Dosis | µg/10µl | 477 | 469 |
| in 48 h resorbierte Dosis | µg | 75,4 | 183,0 |
| in 48 h resorbierte Dosis | % | 15,8 | 39,0 |

Der therapeutische Plasmaspiegel ($C_{ss}$) für die Behandlung von Parkinsonismus liegt bei 1-2 ng/ml, bei den anderen in der Tabelle genannten Indikationen ist der therapeutische Plasmaspiegel geringer.

Der perkutane Fluß (I), der bei einer gegebenen Fläche (A) benötigt wird, um den therapeutischen Plasmaspiegel zu erreichen, läßt sich aus der totalen Körper-Clearance (C1) von LISURID wie folgt errechnen.

$$I = \frac{CL * C_{ss}}{A}$$

Wählt man für ein TDS eine Größe von 50 $cm^2$, so wird bei einer totalen Clearance für LISURID von 65250 ml/h mit einem perkutanen Fluß von 1.3-2.7 $\mu g/cm^2/h$ der gewünschte therapeutische Plasmaspiegel von 1-2 ng/ml erzielt. Die in vitro durch Maushaut erzielten Flüsse von bis zu über 14 $\mu g/cm^2/h$ liegen deutlich über dieser Anforderung. Auch bei realistischer Annahme einer um den Faktor 5 höheren Permeabilität der Maushaut für Lisurid wäre der benötigte transdermale Fluß beim Menschen gegeben.

Die erfindungsgemäßen Ergolin-Derivate enthaltenden Mittel zur transdermalen Applikation können zur Behandlung der gleichen Erkrankungen angewendet werden, wie die vorbekannten, beispielsweise oral oder subcutan zu applizierenden Mittel die Ergolin-Derivate enthalten.

Die nachfolgenden Ausführungsbeispiele dienen zur näheren Erläuterung der Erfindung. In ihnen wurden folgende Handelsprodukte verwendet:

Polyesterfolie von 0,074 mm Dicke (SkotchPak® 1009) des Herstellers 3M; Polypropylenfolie (Celgard® 2500) des Herstellers Celanese, Linerfolie SkotchPak® 1022 und 1360 vom Hersteller 3M; Transferkleber 9871 vom Hersteller 3M, Polyacrylester-Kleber vom Typ Gelva® 2723 des Herstellers Monsanto und Silikonklebstoff vom Typ X-7-4502 des Herstellers Dow Corning.

Beispiel 1

In 100 g einer 50 zigen Lösung von Silikonklebstoff in Benzin werden nacheinander

| | |
|---|---|
| 2,0 g | Lisurid und |
| 10,0 g | Isopropylpalmitat |

unter Rühren gelöst bzw. suspendiert (da die Klebstoffe trübe sind, läßt sich nicht klar entscheiden, ob eine vollständige Lösung vorliegt). Nach Entgasen des Ansatzes über 24 Stunden wird die Lösung/Suspension mittels einer Knife-over-Roll Beschichtungsvorrichtung auf fluorpolymerbeschichtete Polyesterfolie in der Weise aufgetragen, daß nach Entfernung des flüchtigen Lösemittels bei 60-80° C ein gleichmäßiger Film von 100 g/m$^2$ Feststoffauftrag entsteht. Anschließend wird mit einem lichtundurchlässigen Polyester-Abdeckfolie kaschiert. Das so erhaltene Laminat wird mittels einer Stanzvorrichtung in Einzelpflaster von 10 cm$^2$ Fläche geteilt und in einen aluminisierten Beutel luftdicht verpackt. Das Pflaster haftet nach Abziehen der Liner-Folie auf der Haut.

Die Gehaltsbestimmung ergibt eine gleichmäßige Wirkstoffverteilung von 4,4 mg/cm$^2$ im Mittel.

Beispiel 2

In 100 g einer 50 %igen Lösung von Polyacrylester-Klebstoff in Ethylacetat werden nacheinander

| | |
|---|---|
| 1,0 g | Lisurid und |
| 17,5 g | 1,2-Propandiol |

unter Rühren gelöst bzw. suspendiert. Nach Entgasen des Ansatzes über 24 Stunden wird die Lösung/Suspension mittels einer Knife-over-Roll Beschichtungsvorrichtung auf Polyesterfolie in der Weise aufgetragen, daß nach Entfernung der flüchtigen Lösemittel bei 60-80° C ein gleichmäßiger Film von 50 g/m$^2$ Feststoffauftrag entsteht. Anschließend wird mit einer lichtundurchlässigen Polyester Abdeck-Folie kaschiert. Das so erhaltene Laminat wird mittels einer Stanzvorrichtung in Einzelpflaster von 10 cm$^2$ Fläche geteilt und in einen aluminisierten Beutel luftdicht verpackt. Das Pflaster haftet nach Abziehen der Liner-Folie auf der Haut.

Der Gehalt an Ergolin-Derivat beträgt im Mittel 1,1 mg/cm$^2$.

Beispiel 3

Zu 100 g einer 50 %igen Lösung von Polyacrylester-Klebstoff in Ethylacetat werden nacheinander

| | |
|---|---|
| 1.0 g | Tergurid |
| 1,0 g | hochdisperse Kieselsäure und |
| 17,5 g | 1,2-Propandiol mit 10 % 1-Dodecanol |

unter Rühren gelöst bzw. suspendiert. Nach Entgasen des Ansatzes über 24 Stunden wird die Lösung/Suspension mittels einer Knife-over-Roll Beschichtungsvorrichtung auf einen silikonisierten Polyester-Liner in der Weise aufgetragen, daß nach Entfernung der flüchtigen Lösemittel bei 60-80° C ein gleichmäßiger Film von 100 g/m$^2$ Feststoffauftrag entsteht. Anschließend wird mit einer lichtundurchlässigen Polyester-Abdeck-Folie kaschiert. Das so erhaltene Laminat wird mittels einer Stanzvorrichtung in Einzelpflaster von 10 cm$^2$ Fläche geteilt und in aluminisierten Beutel luftdicht verpackt. Das Pflaster haftet nach Abziehen der Liner-Folie auf der Haut.

Der Gehalt an Tergurid liegt bei je 2,2 mg/cm$^2$.

Beispiel 4

Eine lichtundurchlässige Polyesterfolie von 7,4 cm Durchmesser wird mittels Zug und Wärme so verformt, daß eine runde Ausbuchtung von 10 $cm^2$ Fläche entsteht. Diese wird mit 1 ml einer Suspension von

| | |
|---|---|
| 3,0 mg | Protergurid |

in 1,2-Propandiol, welches 10 % Dexpanthenol enthält, gefüllt. Eine Polypropylen-Folie wird am Rand aufgeschweißt. Je nach Druck pro Zeiteinheit liegt die Siegeltemperatur zwischen 70° C und 100° C. Auf die durchlässige Polymerschicht wird Haftkleberfolie transferiert. Das Pflaster wird mit einem Liner versehen und in einem aluminisierten Beutel luftdicht verpackt.

## Patentansprüche

1. Transdermales therapeutisches System zur Verabreichung von Lisurid, 2-Bromlisurid, Tergurid, Protergurid oder deren physiologisch unbedenklichen Salze bestehend aus

   a) einer undurchlässigen Deckschicht, einer an der Deckschicht haftenden, das Ergolinderivat und gegebenenfalls penetrationsverstärkende Mittel enthaltenden, für diese Komponenten durchlässigen Arzneimittelschicht, wobei die Arzneimittelschicht einen Polyacrylat-Hauthaftkleber enthält, so daß die Arzneimittelschicht selbstklebend ist oder von einem Polyacrylat-Hauthaftkleber abgedeckt oder umgeben ist, welcher gegebenenfalls penetrationsverstärkende Mittel enthält, und einer abziehbaren Schutzschicht oder
   b) einer undurchlässigen Deckschicht, einem an oder in der Deckschicht befindlichen, das Ergolinderivat und gegebenenfalls penetrations-verstärkende Mittel enthaltenden Arzneimittelreservoir, einer für diese Komponenten durchlässigen Polymerschicht, einer durchlässigen, gegebenenfalls penetrationsverstärkende Mittel enthaltenden Polyacrylat-Hauthaftkleber-Schicht und einer abziehbaren Schutzschicht,

   wobei die penetrationverstärkenden Mittel Propylenglykol (1,2-Propandiol) und Laurylalkohol sind.

2. Transdermales therapeutisches System nach Anspruch 1, wobei die penetrationsverstärkenden Mittel eine Mischung aus Propylenglykol und Laurylalkohol in einem Gewichtsverhältnis von 9:1 sind.

## Claims

1. A transdermal therapeutic system for administering lisuride, 2-bromolisuride, terguride, proterguride, or their physiologically harmless salts consisting of

   a) an impermeable covering layer, a pharmaceutical layer that adheres to said covering layer and contains the ergoline derivative and, optionally, penetration-enhancing agents and is permeable to these components wherein said pharmaceutical layer contains a polyacrylate skin adhesive so that the pharmaceutical layer is self-adhesive or covered or encompassed by a polyacrylate skin adhesive which may contain penetration-enhancing agents, and a detachable protective layer, or

   b) an impermeable covering layer, a drug reservoir that is located on or in said covering layer and contains the ergoline derivative and, optionally, penetration-enhancing agents, a polymer layer that is permeable to these components, a permeable polyacrylate skin adhesive layer that may optionally contain penetration-enhancing agents, and a detachable protective layer

   wherein propylene glycol (1,2-propanediol) and lauryl alcohol are used as penetration-enhancing agents.

2. The transdermal therapeutic system according to claim 1 wherein a mixture of propylene glycol and lauryl alcohol at a 9 to 1 weight ratio is used as the penetration-enhancing agent.

## Revendications

**1.** Système thérapeutique transdermique pour l'administration de lisuride, de 2-bromolisuride, de terguride, de proterguride ou de leurs sels compatibles physiologiquement, consistant en

a) une couche de recouvrement imperméable, une couche médicamenteuse adhésive à la couche de recouvrement contenant le dérivé d'ergoline et éventuellement des agents intensifiant la pénétration, cette couche médicamenteuse étant perméable pour ces composants, système dans lequel la couche médicamenteuse contient un agent polyacrylate adhésif à la peau de sorte que la couche médicamenteuse est auto-adhésive ou est entourée ou recouverte d'un agent polyacrylate adhésif à la peau qui contient éventuellement des agents intensifiant la pénétration et une couche de protection pouvant être décollée
ou
b) une couche de recouvrement imperméable, un réservoir médicamenteux contenant le dérivé d'ergoline et éventuellement des agents intensifiant la pénétration, se trouvant sur ou dans la couche de recouvrement, une couche polymère perméable pour ces composants, une couche d'agent polyacrylate adhésif à la peau, perméable contenant éventuellement des agents intensifiant la pénétration et une couche de protection pouvant être décollée,

dans lequel les agents intensifiant la pénétration sont le propylène - glycol (1,2-propanediol) et l'alcool laurique.

**2.** Système thérapeutique transdermique selon la revendication 1, dans lequel les agents intensifiant la pénétration sont un mélange de propylène - glycol et d'alcool laurique dans un rapport pondéral de 9:1.